# EUROPÄISCHE PATENTANMELDUNG

(11) **EP 1 895 437 A1**
(43) Veröffentlichungstag der Anmeldung: **05.03.2008**
(21) Anmeldenummer: 06018307.6
(22) Anmeldetag: 01.09.2006
(51) Int. Cl.: G06F 19/00, A61M 5/142

(54) **Medizinische Infusionsgeräte und Verfahren zur Verwaltung solcher Geräte**

(71) Anmelder: F.HOFFMANN-LA ROCHE AG, 4070 Basel (CH); Roche Diagnostics GmbH, 68305 Mannheim (DE)
(72) Erfinder: Lindegger, Stefan, 4932 Lotzwil (CH)
(74) Vertreter: Blum, Rudolf Emil

(57) **Zusammenfassung**

Bei einer tragbaren Infusionspumpe, insbesondere einer Insulinpumpe (1) ist ein Firmwarespeicher (8) vorgesehen, der über eine drahtlose Schnittstelle (3) neu programmierbar ist. Damit lassen sich Insulinpumpen auf einfache Weise mit neuer Firmware versehen.

## Beschreibung

Die Erfindung betrifft ein medizinisches Infusionsgerät, insbesondere ein zur Befestigung und zum Tragen am Körper eines Patienten ausgestaltetes Infusionsgerät und insbesondere eine Insulinpumpe, welches Gerät eine Steuerung und einen Firmwarespeicher aufweist. Ferner betrifft die Erfindung ein Verfahren zur Verwaltung einer Vielzahl von medizinischen Infusionsgeräten.

Infusionsgeräte, die vom Patienten am Körper getragen werden, sind bekannt, insbesondere in der Form von Insulinpumpen für Diabetiker. Solche Geräte weisen eine elektronische Steuerung auf, deren Funktionen durch modellspezifische Software gesteuert wird, die bei der Herstellung des Gerätes in einem Speicher abgelegt wird. Derartige Software wird in der Regel als Firmware bezeichnet.

Der Erfindung liegt die Aufgabe zu Grunde eine verbesserte medizinische Infusionspumpe und insbesondere Insulinpumpe zu schaffen und die Möglichkeit zur Verwaltung einer Vielzahl derartiger Geräte zu schaffen.

Dies wird mit einem medizinischen Infusionsgerät nach Anspruch 1 geschaffen sowie mit einem Verfahren nach Anspruch 6.

Bei einem medizinischen Infusionsgerät gemäss der Erfindung kann eine Neuprogrammierung der Firmware (nachfolgend Firmwareupdate genannt) einfach durch den Kundensupport oder durch den Patienten oder Arzt selber durchgeführt werden, was in einer Arztpraxis oder beim Patienten durchgeführt werden kann. Damit kann ein Firmwareupdate auch für eine grosse Anzahl von bereits in Betrieb befindlichen Infusionsgeräten und insbesondere Insulinpumpen auf einfache Weise erfolgen. Dadurch, dass die Geräte und ihre Firmwareversionen in einer zentralen Datenbank verwaltet werden und von dieser die Firmwareupdates gesteuert und überwacht werden, kann eine optimale Kontrolle der Firmware der sich in Gebrauch befindlichen Geräte bzw. Insulinpumpen erfolgen.

Bevorzugt ist eine Funkschnittstelle oder eine Infrarotschnittstelle für die drahtlose Übermittlung von Firmwareversionen an das Gerät. Bevorzugterweise erfolgt dies über das Internet. Bei einer Ausführungsart kann das Gerät selbständig eine Funktionsprüfung der neuen Firmware durchführen, bevor diese in den eigentlichen Firmwarespeicher geladen wird und die Steuerung des Gerätes übernimmt. Die Funktionsprüfung kann auch erst im Firmwarespeicher erfolgen.

Im Folgenden werden Ausführungsbeispiele der Erfindung anhand der Zeichnungen näher erläutert. Dabei zeigt
Figur 1 grob schematisch eine erste Ausführungsform der Erfindung; und
Figur 2 eine weitere Ausführungsform, insbesondere zur Erläuterung des erfindungsgemässen Verfahrens.

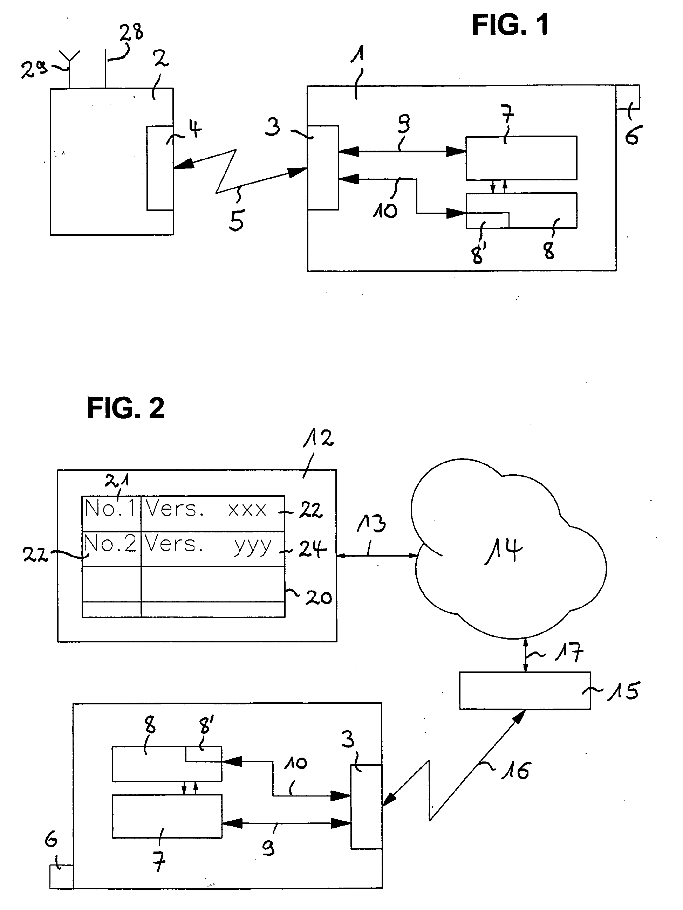

Figur 1 zeigt grob schematisch ein medizinisches Infusionsgerät 1, insbesondere eine am Körper eines Patienten tragbare Insulinpumpe. Derartige Insulinpumpen sind bekannt und müssen hier mit Bezug auf ihre Funktionen nicht näher erläutert werden. Diese Insulinpumpen sind zur Aufnahme eines Vorrates von Insulin ausgestaltet, welches über einen Anschluss 6 an eine Infusionsleitung abgegeben wird, die zu einer im Körper des Patienten befindlichen Infusionsnadel führt. Die Insulinpumpe gibt programmgesteuert und unter Berücksichtigung von Eingabewerten durch den Patienten während vieler Stunden die jeweils benötigte Insulinmenge an den Patienten ab. Die Abgabe erfolgt durch einen Pumpenmotor, der einen Kolben bewegt. Der nicht dargestellte Pumpenmotor wird durch die in Figur 1 mit 7 bezeichnete Steuerung des Gerätes 1 betrieben, was hier, da bekannt, nicht näher erläutert wird. Weiter bekannt ist es, dass in der Steuerung 7 eine Firmware eingebettet ist, welche das Verhalten der Steuerung bestimmt. In der Figur ist dies zur Verdeutlichung durch einen Firmwarespeicher 8 dargestellt, welcher mit der eigentlichen Steuerung kommuniziert. Der Firmwarespeicher 8 kann z.B. ein Flash-Speicher oder ein EEPROM-Speicher sein. Der Begriff der Firmware ist bekannt. Diese nimmt als Software in der Hardware eine Mittelstellung zwischen Software und Hardware ein. Sie ist modellspezifisch bzw. funktioniert nicht auf anderen Gerätemodellen. Die Firmware wird bei der Herstellung des Gerätes gespeichert und ohne diese Firmware ist das Gerät nicht funktionsfähig. Gemäss der Erfindung ist das Gerät 1 nun mit einer Schnittstelle 3 versehen, die auf drahtlosem Weg Daten mit einem weiteren Gerät austauschen kann, was durch den Pfeil 5 und das weitere Gerät 2 dargestellt ist. Durch die Schnittstelle 3 ist nun ein Firmwareupdate, d.h. eine vollständige oder teilweise Erneuerung der im Gerät 1 befindlichen Firmware durchführbar. Dies kann derart erfolgen, dass die zu ladende Firmware von der Schnittstelle 3 an die Steuerung 7 und von dort in den Firmwarespeicher 8 abgegeben wird, was durch den mit dem Pfeil 9 angedeuteten Datenpfad möglich ist. Andererseits ist es auch möglich, was durch den Pfad 10 angedeutet ist, dass Firmwareupdates von der Schnittstelle 3 direkt in den Speicher 8 abgegeben werden. Nach dem Laden und der Verifizierung der neuen Firmware, was noch näher erläutert wird, erfolgt der Betrieb des Gerätes 1 mit der neuen Firwareversion. Durch diese können Fehler in der bestehenden Firmware behoben werden und/oder es können neue Funktionalitäten des Gerätes 1 eingeführt werden.

Die Übertragung der Pumpensoftware bzw. Firmware erfolgt drahtlos und bevorzugterweise durch Datenübertragung in Form von elektromagnetischen Wellen oder Lichtimpulsen, insbesondere Infrarotkommunikation. Bei einer Funkübertragung ist insbesondere eine Übertragung gemäss dem Bluetooth-Standard bevorzugt. Da die Programmierung des Firmwareupdates drahtlos erfolgt, benötigt die Insulinpumpe kein Kabel zur Datenübertragung. Die eigentliche Datenübertragung kann über eine möglichst sichere und insbesondere verschlüsselte Verbindung erfolgen, dabei können herstellerspezifische Protokolle des Insulinpumpenherstellers oder Standardprotokolle verwendet werden.

Als Sende- bzw. Programmiergerät kann insbesondere ein Desktop oder Laptop-PC verwendet werden, ein Pocketorganizer, ein Mobiltelefon oder ein anderes geeignetes handelsübliches Gerät, das zu einem drahtlosen Datenaustausch fähig ist. Alternativ dazu kann das Gerät 2 auch ein spezifisch für diese Aufgabe hergestelltes Programmiergerät sein. Die an das medizinische Gerät 1 zu übermittelnde Firmware kann durch eine Kabelverbindung 28 in das Gerät 2 eingespiesen werden oder dieses Gerät 2 kann die Firmware ebenfalls drahtlos empfangen, was durch das Antennensymbol 29 angedeutet ist. So kann zum Beispiel das Gerät 2 ein Mobiltelefon sein, das sich im Besitz des Patienten befindet, der die Insulinpumpe 1. trägt. Auf diese Weise kann der Patient über das Gerät 2 ein Firmwareupdate vom Pumpenhersteller empfangen und dieses an die Insulinpumpe 1 übertragen. Das Gerät 2 kann ein Computer in einer Arztpraxis sein, wodurch der Arzt das Firmwareupdate empfängt und dieses beim nächsten Patientenbesuch an die Infusionspumpe 1 des Patienten überträgt.

Bei einer besonderen Ausführungsform der Erfindung wird die neue oder geänderte Firmware zunächst in einen speziellen Firmwarespeicher 8' gespeichert, welcher, wie in der Figur 1 angedeutet, Teil des normalen Firmwarespeichers 8 sein kann oder allenfalls ein separater Speicher. Jedenfalls kommuniziert dieser Speicher 8' auch mit der Steuerung. Das Laden in den Speicher 8' kann über beide Pfade 9 oder 10 erfolgen. Das Gerät 1 besitzt dadurch zunächst weiterhin die bestehende Firmware sowie die neu in den Speicherteil 8' geladene Firmware. In diesem Speicherteil 8' kann nun, insbesondere in Zusammenarbeit mit der Steuerung 7, eine Prüfung der neuen Firmware erfolgen. Diese Prüfung kann sich allenfalls nur darauf beziehen, dass geprüft wird, ob die Firmware vollständig und fehlerfrei übertragen und gespeichert worden ist und eine Versionsnummer beinhaltet, welche höher als die Versionsnummer der bestehenden Firmware ist und als eine gültige Versionsnummer des Pumpenherstellers erkannt werden kann. Da die Firmware vorgängig schon herstellerseitig geprüft und allenfalls erst nach einer Verifikation freigegeben worden ist, kann die Funktionsprüfung im Gerät entsprechend reduziert erfolgen.

Bei einem anderen Ausführungsbeispiel kann die Überprüfung der neu gespeicherten Firmware weiter gehen, z.B. indem diese in Zusammenarbeit mit der Steuerung Funktionstest unterworfen wird. So kann mit der Firmware im Speicherteil 8' ein umfangreicher Funktionstest der Pumpe durchgeführt werden, ohne dass dies allerdings zu entsprechenden tatsächlichen Insulinabgaben der Pumpe 1 während des Testbetriebes führt. Die Steuerung kann aber ermitteln, ob aufgrund der jeweiligen Situation die durch die neue Firmware bestimmten Befehle sinnvoll sind bzw. sich innerhalb vorgegebener Grenzen bewegen. Die Pumpe 1 wird also in dieser Phase weiterhin durch die bisherige Firmware betrieben und daneben erfolgt ein Testbetrieb der neuen Firmware, welche dadurch verifiziert werden kann und wodurch insbesondere festgestellt werden kann, ob die neue Firmware auf dieser individuellen Pumpe 1 richtig funktioniert und jedenfalls keine Pumpenfunktion bewirkt, welche für den Patienten schädlich oder gefährlich ist.

Bevorzugt wird erst nach der einfachen oder nach der beschriebenen umfangreicheren Prüfung mit einem positiven Resultat, das die korrekte Funktion der neuen Firmware bestätigt, die neue Firmware als die Funktion der Pumpe 1 bestimmende Firmware übernommen bzw. die bisherige Firmware überschrieben oder es erfolgt eine Umschaltung der Speicher, so dass die Steuerung mit dem die neue Firmware enthaltenden Speicher betrieben wird.

Figur 2 zeigt eine Darstellung, worin wiederum ein Gerät 1 dargestellt ist, welches dem Gerät 1 von Figur 1 entspricht und insbesondere ebenfalls eine Insulinpumpe ist und worin gleiche Bezugszeichen wiederum gleiche Elemente benennen. Die Schnittstelle 3 der Infusionspumpe 1 steht hierbei mit dem Internet in Verbindung, insbesondere z.B. über einen Wireless Access Point 15, was durch den Pfeil 16 angedeutet ist. In diesem Fall handelt es sich um eine Funkverbindung vom Wireless Access Point 15 zum Gerät 1. Der Wireless Access Point 15 ist ein Zugangspunkt zum Internet und über eine Datenverbindung 17 mit dem Internet 14 verbunden. Ebenfalls mit dem Internet 14 über eine Datenverbindung 13 verbunden ist ein Rechner 12, auch welchem eine Datenbankapplikation 20 in Betrieb ist. Diese Datenbank 20 verwaltet Firmwareinformationen für eine Vielzahl von Geräten 1. Über das Internet kann diese Datenbank mit einzelnen der Vielzahl von Geräten 1 in Kontakt treten und auf die bereits geschilderte Weise ein Firmwareupdate durchführen. Die Datenbank erlaubt dabei die Verwaltung und die Übersicht über sämtliche Firmwareversionen, die in den einzelnen Geräten 1 in Verwendung sind. Dazu ist in der Datenbank 20 jedes Gerät mit einer spezifischen Identifikation erfasst, was im Beispiel von Figur 2 mit den Angaben No. 1 im Feld 21 und No. 2 im Feld 23 der Datenbank angedeutet ist. Mit anderen Worten für jedes verwaltete Gerät ist dessen Identifikationsnummer in der Datenbank enthalten. Zugehörig zu dieser Identifikationsnummer ist die entsprechende Firmwareversion, welche im jeweiligen Gerät im Einsatz ist, in der Datenbank enthalten. In der Figur ist das durch die Felder 22 und 24 angedeutet, in welcher jeweils die Versionsnummer XXX bzw. YYY zu dem jeweiligen Gerät angegeben ist. Natürlich kann die Datenbank viele weitere Informationen über das jeweilige Gerät enthalten, so insbesondere die Versionsgeschichte der verschiedenen nacheinander eingesetzten Firmwares, Wartungsdaten und/oder Reparaturdaten der Geräte oder sonstige Besonderheiten. Auf diese Weise kann der Hersteller der Geräte 1 durch das drahtlose Firmwareupdate und die Datenbank vollständige Informationen über die Firmwarekonfiguration jedes im Einsatz befindlichen Gerätes besitzen. Der Hersteller kann diese Information auch dem jeweiligen den Patienten bzw. das Gerät 1 betreuenden Arzt zugänglich machen oder allenfalls dem Patienten. Der Kontakt des Gerätes 1 mit der Datenbank kann dabei auf zufällige Weise aufgenommen werden, jedes Mal wenn sich das Gerät 1 bzw. der Patient im Bereich eines Wireless Access Points bzw. eines Wireless Local Area Netzwerkes befindet, in dem das Gerät 1 dann Kontakt mit der Datenbank aufnimmt. Natürlich kann der Kontakt mit der Datenbank auch dann hergestellt werden, wenn sich der Patient beim Arzt befindet und dieser über das in Figur 1 gezeigte Gerät 2 verfügt, welches ebenfalls mit der Datenbank kommunizieren kann, sei dies über das Internet oder über eine Telefonverbindung. Anstelle eines Wireless Access Points 15 kann auch das Mobiltelefon des Patienten treten, welches ebenfalls in Kontakt mit der Datenbank treten kann. Damit ist die Insulinpumpe bzw. der Patient jederzeit in der Lage, selbständig einen sicheren Firmwareupdate durchzuführen, falls ein solcher möglich ist bzw. falls eine neue Firmwareversion existiert. Ist eine solche nicht vorhanden, so wird dies bei der Kontaktaufnahme festgestellt und es erfolgt kein Firmwareupdate.

## Patentansprüche

1. Medizinisches Infusionsgerät (1), insbesondere ein zur Befestigung und zum Tragen am Körper eines Patienten ausgestaltetes Infusionsgerät und insbesondere eine Insulinpumpe, welches Gerät eine Steuerung (7) und einen Firmwarespeicher (8) aufweist, **dadurch gekennzeichnet, dass** der Firmwarespeicher (8) über eine drahtlose Schnittstelle (3) des Gerätes programmierbar ist.

2. Medizinisches Infusionsgerät nach Anspruch 1 **dadurch gekennzeichnet, dass** die Schnittstelle eine Funkschnittstelle ist, insbesondere eine Funkschnittstelle nach dem Bluetooth^{®}-Standard ist.

3. Medizinisches Infusionsgerät nach Anspruch 1, **dadurch gekennzeichnet, dass** die Schnittstelle eine optische Schnittstelle, insbesondere eine InfrarotSchnittstelle ist.

4. Medizinisches Infusionsgerät nach einem der Ansprüche 1 bis 3, **dadurch gekennzeichnet, dass** das Gerät (1) zur Verbindungsaufnahme mit dem Internet (14) über einen Wireless Access Point (15) und zum Datenaustausch über das Internet zur Programmierung des Firmwarespeichers mit Daten, welche über das Internet übermittelt worden sind, ausgestaltet ist.

5. Medizinisches Infusionsgerät nach einem der Ansprüche 1 bis 4, **dadurch gekennzeichnet, dass** das Gerät (1) mit einem weiteren Speicher (8') versehen ist, in welchen Firmenupdates ladbar sind, dass das Gerät ein Prüfmittel (7) aufweist, mittels dem die im weiteren Speicher geladene Firmware prüfbar ist, und dass die Übertragung der im weiteren Speicher (8') gespeicherten Firmware in den Firmwarespeicher (8), oder eine Umschaltung der Speicher für den Betrieb der Steuerung mit der neuen Firmware im weiteren Speicher (8'), abhängig vom Resultat der Prüfung vornehmbar ist.

6. Verfahren zur Verwaltung einer Vielzahl von medizinischen Geräten (1) nach Anspruch 1, **dadurch gekennzeichnet, dass** jedes der verwalteten Geräte in einer Datenbank (20) mit einer individuellen Kennung (21) erfasst ist und für jedes Gerät die aktuelle und allenfalls frühere Firmwareversionen (22) erfasst ist bzw. sind, dass über die Datenbank die Erneuerung der Firmware einzelner Geräte und/oder von Gruppen von Geräten und/oder aller Geräte ausgelöst und/oder überwacht wird, und dass nach einer erfolgreichen Erneuerung der Firmware dies in der Datenbank vermerkt wird.

7. Verfahren nach Anspruch 6, **dadurch gekennzeichnet, dass** die Geräte mit der Datenbank über das Internet (14) kommunizieren.

8. Verfahren nach Anspruch 7, **dadurch gekennzeichnet, dass** ein Gerät, das in den Bereich eines Internet Zuganges, insbesondere eines Wireless Access Points (15), gelangt, Verbindung mit der Datenbank aufnimmt, dass Gerät und Datenbank prüfen, ob für das Gerät eine Erneuerung der Firmware vorgesehen ist und, falls dies der Fall ist, dass die Datenbank die Erneuerung der Firmware durchführt.
